# FASCICULE DE BREVET EUROPEEN

(11) **EP 3 365 479 B1**
(45) Date de publication et mention de la délivrance du brevet: **04.09.2019**
(21) Numéro de dépôt: 16798545.6
(22) Date de dépôt: 18.10.2016
(51) Int. Cl.: C25D 17/08, A61C 19/00, C25D 11/00, A61C 8/00, C25D 11/26

(54) **DISPOSITIFS DE SUPPORT POUR SUPPORTER DES IMPLANTS OU DES PROTHESES**
STÜTZVORRICHTUNGEN ZUR STÜTZE EINES IMPLANTATS ODER EINER PROTHESE
SUPPORT DEVICES FOR SUPPORTING IMPLANTS OR PROSTHESES

(30) Priorité: 22.10.2015 FR 1560110
(43) Date de publication de la demande: 29.08.2018
(73) Titulaire: Les Laboratoires Osteal Medical, 95957 Roissy Aeroport CDG (FR)
(72) Inventeur: BOUCHOT, Daniel, 77186 Noisiel (FR); TISSERAND, Thierry, 60128 Plailly (FR)
(74) Mandataire: CAPRI
(86) Numéro de dépôt international: PCT/FR2016/052683
(87) Numéro de publication internationale: WO 2017/068273

(56) Documents cités:
- EP-A1- 1 129 232
- CN-U- 203 834 046
- FR-A1- 2 875 420
- JP-A- H10 176 300
- KR-B1- 100 831 341
- US-A1- 2014 331 924

## Description

La présente invention concerne des outils, ustensiles, ancillaires ou plus généralement des dispositifs de support pour supporter des implants ou des prothèses, tels que des implants dentaires afin de les manipuler lors d'un traitement de surface. Avec de tels dispositifs de supports, les implants peuvent subir un procédé de greffage de polymère bioactif, tel que du PolyNaSS (polystyrène sulfonate de sodium). Bien que la présente invention soit plus particulièrement destinée aux implants dentaires dans le but d'un greffage de polymère bioactif, elle peut également être mis en oeuvre avec d'autres types d'implants dans le but d'un autre traitement de surface ou autre. Il est connu du document JP H10 176300 un élément de support d'articles destinés à recevoir un revêtement par électrophorèse, ledit élément comprenant une barrette et plusieurs broches raccordées à la barrette.

Dans l'art antérieur, on connaît le document EP2032663 qui décrit un procédé de greffage de polymère bioactif sur un matériau prothétique en titane ou alliage de titane. Ce procédé préconise trois étapes successives, à savoir :
- la génération d'espèces actives donneuses de radicaux libres à la surface du matériau prothétique,
- la génération de radicalaire à la surface du matériau prothétique par réactions thermiques, et
- la mise en présence du matériau prothétique avec au moins un monomère porteur d'une fonction permettant une polymérisation radicalaire. La polymérisation radicalaire dudit monomère permettant la formation d'un polymère bioactif en l'absence d'oxygène.

Par conséquent, le procédé de ce document EP2032663 est plus particulièrement orienté sur les réactions et interactions physiques ou chimiques permettant de synthétiser le polymère bioactif directement à partir de la surface d'un implant. Cela donne au polymère bioactif la caractéristique d'être greffée de manière permanente sur l'implant. Le polymère bioactif est de préférence du PolyNaSS et le type d'oxydation de l'implant est de préférence une oxydation chimique.

Bien que le procédé du document EP2032663 trace les grandes lignes à suivre pour réaliser le procédé de greffage en laboratoire à petite échelle, il ne donne absolument aucune indication quant à l'application de ce procédé de greffage de manière industrielle à grande échelle.

La présente invention a précisément pour but d'utiliser ce procédé de greffage de polymère bioactif de manière industrielle à grande échelle. En d'autres termes, il s'agit de mettre en oeuvre ce procédé de greffage dans un milieu industriel afin de traiter de grandes séries d'implants à la fois. Alors que l'oxydation chimique est mise en avant dans le procédé de greffage du document EP2032663, il s'est rapidement avéré que ce type d'oxydation (chimique) est tout à fait inapproprié, voire dangereux, dans un environnement industriel. La présente invention s'est ainsi tournée vers l'oxydation anodique, qui n'est pas mentionnée dans le document EP2032663.

C'est précisément en vue de cette industrialisation du procédé de greffage qu'il a été développé plusieurs dispositifs de support permettant une manipulation aisée des implants.

Dans ce cadre, la présente invention propose tout d'abord un élément de support d'implants, tels que des implants dentaires, comprenant une barrette et plusieurs broches raccordées à la barrette et disposées parallèlement les unes aux autres, chaque broche définissant une extrémité libre pourvue de moyens de réception aptes à coopérer avec l'implant pour maintenir l'implant sur les moyens de réception de la broche, la barrette comprenant au moins une extrémité de montage pour le montage de la barrette sur un dispositif de support pour ainsi former une structure de support.

Ainsi, l'élément de support d'implants présente une configuration générale similaire à celle d'un peigne avec une barrette commune à partir de laquelle se dressent des dents parallèles. L'élément de support d'implants peut être saisi manuellement ou mécaniquement au niveau de sa barrette, et plus particulièrement au niveau de son extrémité de montage. Cet élément de support d'implants constitue ainsi un élément de base qui peut être monté sur plusieurs dispositifs de support, afin de créer une structure de support complexe.

Selon l'invention, chaque broche est pourvue d'un système de retrait pour retirer l'implant des moyens de réception sans venir en contact avec une partie exposée de l'implant. Selon un mode de réalisation avantageux, le système de retrait comprend une bague rotative montée sur la broche, l'implant en prise avec les moyens de réception venant en contact de la bague rotative, de sorte qu'une rotation de la bague rotative a pour effet de désolidariser l'implant de ses moyens de réception. De préférence, chaque broche comprend une embase raccordée à la barrette, l'embase étant surmontée d'une tigelle dont l'extrémité libre est pourvue d'un filetage formant les moyens de réception, la bague rotative étant engagée autour de la tigelle en prenant appui sur l'embase, l'extrémité libre filetée faisant saillie hors de la bague rotative afin de permettre un vissage de l'implant sur l'extrémité libre filetée en venant en butée sur la bague rotative. Ainsi, la rotation libre de la bague rotative autour de la tigelle a pour effet de dévisser l'implant de l'extrémité libre filetée, de sorte que l'implant peut se libérer de son élément de support. Ce sont les vibrations de la bague rotative qui sont transmises lors de sa rotation à l'implant qui est en butée sur la bague rotative. Il faut savoir qu'il n'est pas nécessaire que l'implant soit entièrement engagé sur l'extrémité libre filetée : il suffit que l'implant soit à peine vissé sur l'extrémité libre filetée pour le maintenir en position, étant donné qu'il vient en butée sur la bague rotative. De ce fait, une légère rotation de la bague rotative suffit à dévisser l'implant de l'extrémité libre filetée.

Selon un autre aspect pratique, la barrette supporte une douzaine de broches avec un écart entre broches de l'ordre de 1 à 2 cm. D'autre part, la barrette peut présenter une section transversale de forme cylindrique circulaire tronquée de manière à former un méplat longitudinal qui est disposé de manière opposée aux broches. Ainsi, ce méplat longitudinal sert de surface d'appui permettant de disposer l'élément de support debout sur une surface plane.

L'invention définit également une platine de montage d'éléments de support d'implants, comprenant plusieurs logements de montage dans lesquels sont engagées les extrémités de montage des barrettes d'éléments de support d'implants tel que défini ci-dessus. La platine de montage avec ses éléments de support d'implants constitue ainsi une structure de support complexe qui permet une manipulation aisée de plusieurs éléments de supports d'implants à la fois.

Selon un aspect avantageux, les broches sont disposées tête-bêche les unes par rapport aux autres. D'autre part, la platine de montage peut également comprendre un manche de préhension amovible.

Cette platine de montage peut par exemple être utilisée au cours du procédé de greffage, plus particulièrement lors des étapes de décapage, d'anodisation et de rinçage.

La présente invention définit également une plaque de montage d'éléments de support d'implants, comprenant plusieurs rails de montage dans lesquels sont engagées les barrettes d'éléments de support d'implants tel que défini ci-dessus. A nouveau, cette plaque de montage équipée d'éléments de support d'implants constitue une structure de support complexe qui peut être utilisée lors du procédé de greffage, particulièrement lors de l'étape de polymérisation.

Avantageusement, les barrettes sont engagées par coulissement à partir d'une extrémité ouverte d'accès, les broches pointant parallèlement dans la même direction. Par ailleurs, la plaque de montage peut également comprendre des moyens de fixation disposés sur une face opposée aux rails de montage pour fixer la plaque sur un chariot élévateur.

Toujours dans le cadre de l'invention, il est aussi défini un panier de lavage comprenant une poutre centrale formant des logements de montage dans lesquels sont engagées les extrémités de montage des barrettes d'éléments de support d'implants tel que défini ci-dessus. A nouveau le panier de lavage et ses éléments de support d'implants constituent une structure de support qui peut être utilisée lors du procédé de greffage, et plus particulièrement lors des étapes de lavage et de séchage.

Selon un aspect pratique, les éléments de support d'implants peuvent être disposés de part et d'autre de la poutre de manière équilibrée, avantageusement avec une inclinaison. En outre, la poutre centrale peut être pourvue d'une anse de préhension.

On vient de voir que l'élément de support d'implants constitue l'élément de base qui peut être associé à d'autres éléments, tels que la platine, la plaque ou le panier pour constituer des srtuctures de support complexes qui seront utilisées lors de certaines étapes du procédé de greffage. Il est à nouveau rappelé que ces éléments sont plus particulièrement adaptés à être utilisés lors d'un procédé de greffage de polymère bioactif, mais ils peuvent également être utilisés pour d'autres types de procédé de traitement.

L'invention sera maintenant plus amplement décrite en référence aux dessins joints donnant plusieurs modes de réalisation de l'invention.

Sur les figures :
La figure 1 est une vue très schématique en bloc diagramme représentant les différentes étapes du procédé de greffage selon l'invention,
La figure 2a est une vue en perspective d'un élément de support d'implant selon l'invention,
La figure 2b est une vue de l'élément de support d'implants de la figure 2a avec des implants montés dessus,
La figure 2c est une vue en coupe transversale verticale agrandie à travers un implant monté sur une broche de l'élément de support d'implants des figures 2a et 2b,
La figure 3 est une vue en perspective d'une platine de montage d'éléments de support d'implants qui a déjà reçu trois éléments de supports d'implants et qui est prêt à en recevoir un quatrième,
La figure 4 est une vue en perspective d'un récipient rempli de gaz inerte dans lequel est déposée la platine de montage avec ses éléments de support d'implants,
La figure 5a est une vue en perspective schématique d'une enceinte mise en oeuvre pour l'étape de polymérisation,
La figure 5b est une vue en perspective agrandie d'un détail de la figure 5a,
La figure 6 est une vue en perspective d'une plaque de montage dans laquelle les éléments de support d'implants sont reçus par coulissement,
La figure 7 est une vue en plan schématique de la station de polymérisation disposée à l'intérieur de l'enceinte de polymérisation,
Les figures 8a à 8e représentent différents modes de réalisation d'une cuve de polymérisation contenant le monomère,
Les figures 9a et 9b sont des vues en perspective d'un autre mode de réalisation de la station de polymérisation,
Les figures 10a et 10b représentent deux variantes de cuves recevant le dispositif des figures 9a et 9b,
La figure 11 est une vue en perspective d'un panier de lavage selon l'invention,
La figure 12a représente un étrier pour l'anodisation de deux implants fémoraux de hanche,
La figure 12b représente un implant fémoral de hanche disposé dans un récipient rempli de gaz inerte,
La figure 12c représente une réglette sur laquelle sont montés les implants fémoraux de hanche,
La figure 12d représente deux réglettes engagées dans des rails de montage de l'ascenseur de la station de polymérisation, et
La figure 12e représente un casier de lavage pour des implants fémoraux de hanche.

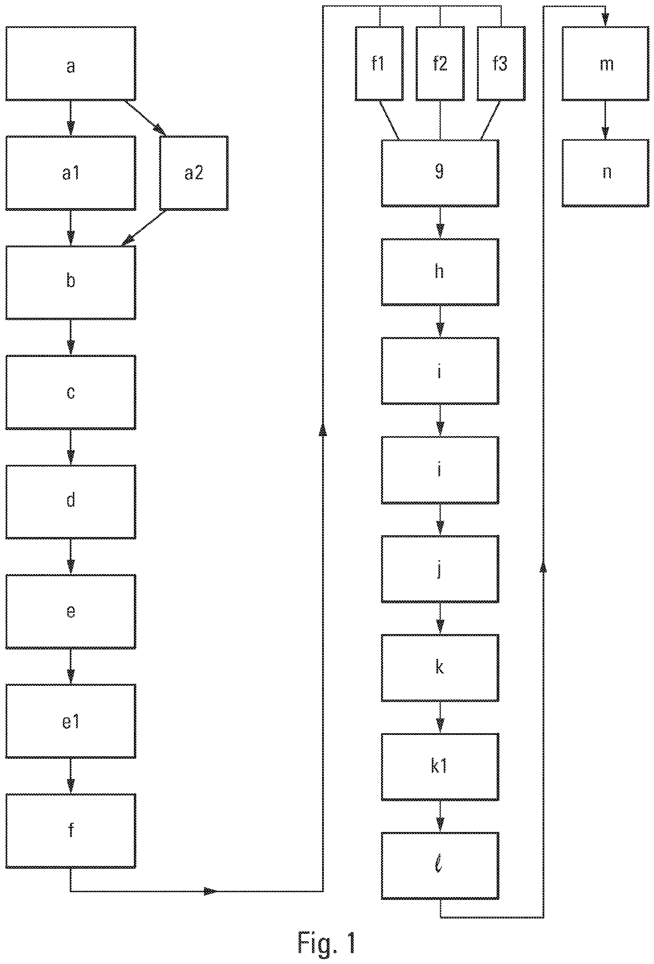

On se référera tout d'abord à la figure 1 pour décrire les différentes étapes successives du procédé de greffage d'un polymère bioactif, tel que du PolyNaSS (polystyrène sulfonate de sodium), sur des implants notamment en titane ou en alliage de titane, afin de réaliser un revêtement antiadhésif ou « anti-accroche » sur lequel les bactéries et autres agents infectieux glissent de sorte qu'ils ne peuvent pas s'y développer.

Les étapes majeures successives sont les suivantes :
a) Monter des implants sur une structure de support d'implants : on verra ci-après un élément de support pour implants dentaires et un étrier de support pour implants fémoraux de hanche.
b) Plonger les implants (montés sur leur support) dans un bain d'acide, tel que de l'acide nitrique et/ou fluorhydrique, pour les décaper : le temps de trempage peut être de l'ordre de 30 s à 1 minute.
c) Rincer les implants, par exemple à l'eau,
d) Plonger les implants (montés sur leur support) dans un bain d'anodisation, par exemple à base d'acide orthophosphorique, pour les anodiser et ainsi créer des peroxydes de titane en surface: le temps de trempage peut être de l'ordre de 10s à 1 minute,
e) Rincer les implants, par exemple à l'eau,
f) Introduire les implants (encore montés sur leur support, ou un autre support, ou sur aucun support) dans une enceinte de polymérisation étanche remplie de gaz inerte, tel que l'argon : un autre gaz inerte peut aussi être utilisé.
g) Monter les implants (montés sur leur support, ou un autre support, ou sur aucun support) sur un ascenseur installé dans l'enceinte étanche,
h) Actionner l'ascenseur pour plonger les implants dans un bain de polymérisation, par exemple de monomère, comme le styrène sulfonate de sodium (NaSS), présent dans l'enceinte,
i) Soumettre le bain de polymérisation à un catalyseur de polymérisation, par exemple thermique ou UV, pour synthétiser du polymère bioactif à la surface des implants, et ainsi obtenir un implant revêtu d'une couche de polymère greffé, par exemple du PolyNaSS,
j) Remonter l'ascenseur pour extraire les implants revêtu du bain de polymérisation,
k) Retirer les implants de l'ascenseur,
l) Extraire les implants de l'enceinte étanche,
m) Laver les implants, par exemple par aspersion d'eau pure, pour les débarrasser de l'excès de polymère bioactif non greffé,
n) Sécher les implants greffés.

Outre ces étapes majeures, le procédé définit également des étapes intermédiaires, secondaires et/ou optionnelles qui améliorent encore davantage les étapes majeures ou permettent une manipulation plus aisée d'implants particuliers, comme par exemple des implants dentaires ou encore des implants fémoraux de hanche. On peut notamment citer les étapes suivantes.

Pour les implants dentaires :
- une étape intermédiaire a1- entre l'étape a- et l'étape b-, consistant à monter plusieurs implants sur des éléments de support, eux-mêmes montés sur une platine de support, comprenant avantageusement un manche de préhension amovible, la platine avec ses éléments de support d'implants constituant une structure de support d'implants,
- les implants, lors des étapes b- à f-, sont manipulés au moyen de la platine de support, avec les éléments de support d'implants montés dessus,
- une étape intermédiaire e1- entre l'étape e- et l'étape f-, consistant à placer la platine de support avec ses éléments de support d'implants dans un récipient rempli de gaz inerte, tel que l'argon, avantageusement pourvu d'un couvercle étanche, le récipient étant ensuite introduit lors de l'étape f- dans l'enceinte étanche remplie de gaz inerte, tel que l'argon, le récipient étant alors ouvert pour en extraire la platine de support avec ses éléments de support d'implants,
- une étape intermédiaire f1- entre l'étape f- et l'étape g-, consistant à retirer les éléments de support d'implants de la platine de support, puis à monter les éléments de support d'implants sur une plaque de support qui est ensuite montée sur l'ascenseur, ou, e, variante, une étape intermédiaire f2-entre l'étape f- et l'étape g-, cette étape f2- consistant à monter sur des tiges axiales verticales (C) de l'ascenseur (L') qui sont entraînées en rotation dans le bain de polymérisation, soit la platine de support (B), soit les éléments de support (S).
- une étape intermédiaire k1- entre l'étape k- et l'étape l-, consistant à retirer les éléments de support d'implants de l'ascenseur, puis à monter les éléments de support d'implants sur une poutre centrale formant des logements de montage pour les éléments de support d'implants afin de former un panier le lavage, qui est ensuite extrait de l'enceinte. Le panier de lavage peut également être constitué à la sortie de l'enceinte étanche.
- lors des étapes m- et n-, les éléments de support d'implants sont configurés sous la forme du panier de lavage.

Pour les implants fémoraux de hanche :
- une étape intermédiaire a2- entre l'étape a- et l'étape b-, consistant à monter plusieurs implants (H) sur un étrier de support (Th), les implants (H), lors des étapes b- à f-, étant manipulés au moyen de l'étrier de support (B),
- une étape intermédiaire f3- entre l'étape f- et l'étape g-, consistant à monter les implants côte à côte sur une réglette qui est ensuite montée sur l'ascenseur,
- lors de l'étape l-, les implants sont sur la réglette,
- lors des étapes m- et n-, les implants sont disposés sur un casier de lavage.

A titre d'exemple, ce procédé de greffage de polymère actif sera maintenant mis en oeuvre sur des implants dentaires pour lesquels il a été développé des outils, accessoires ou ancillaires permettant une manipulation plus aisée, plus rapide et à grande échelle.

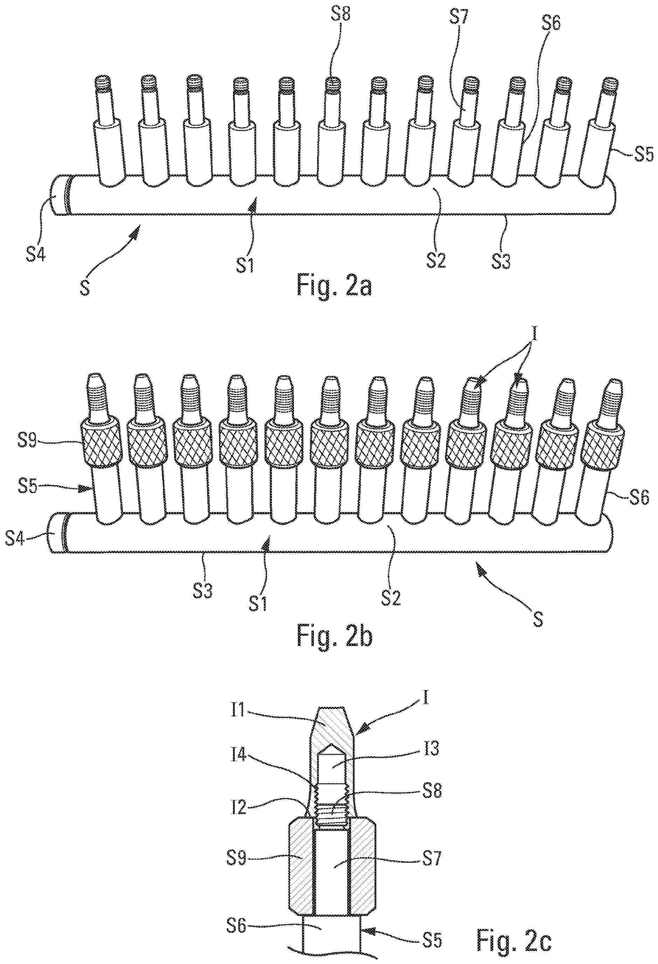

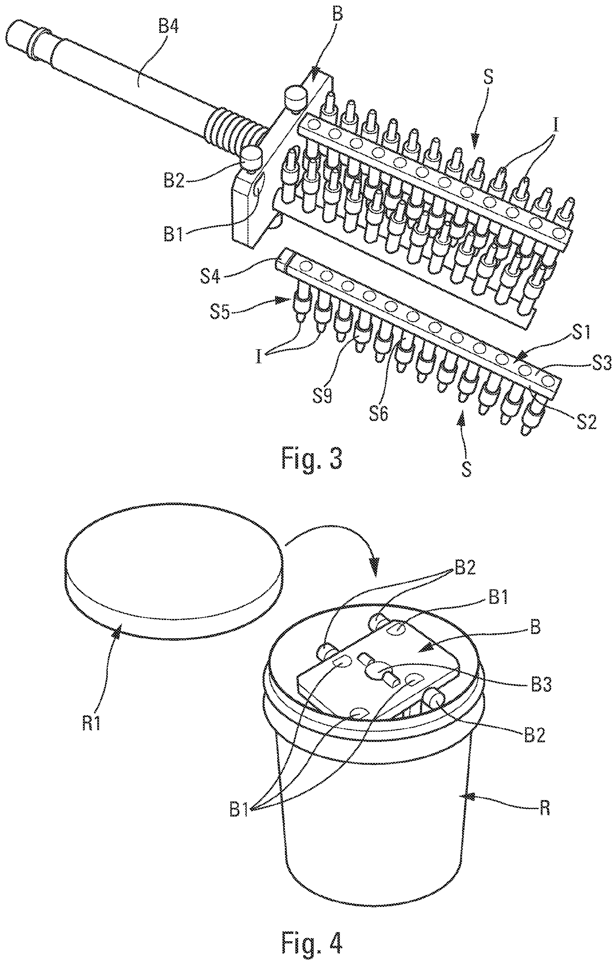

En se référant aux figures 2a, 2b et 2c, on peut voir un élément de support S pour des implants, tels que des implants dentaires I. Un type d'implants I est plus particulièrement visible en coupe sur la figure 2c. On peut voir que l'implant I comprend une tête I1 à son extrémité supérieure et un bord inférieur annulaire I2 à son extrémité opposée. L'implant I définit un intérieur creux I3 présentant une paroi filetée I4. Il s'agit là d'une conception tout à fait classique pour un implant dentaire. L'élément de support S comprend une barrette S1 sur laquelle sont montées plusieurs broches S5 qui s'étendent avantageusement parallèlement les unes aux autres. On peut par exemple prévoir douze broches S5 disposées de manière alignée et parallèle sur une barrette S1 avec un écart entre l'axe de broche de l'ordre de 1 à 2 cm. Selon un mode de réalisation avantageux, la barrette présente une section transversale cylindrique circulaire tronquée à sa base de manière à former un méplat longitudinal qui est disposé de manière opposée aux broches S5. Ceci est plus visible sur la figure 3. La barrette S1 comprend au moins une extrémité de montage pour le montage de la barrette sur une structure de support, qui sera définie ci-après. Cette extrémité de montage S4 peut même présenter un logement creux. Quant aux broches S5, elles comprennent chacune une embase S6 raccordée à la barrette S1, une tigelle S7 montée sur l'embase S5 en définissant un diamètre inférieur et comprenant une extrémité libre pourvue d'un filetage S8. Un épaulement est ainsi formé au niveau de l'extrémité supérieure de l'embase autour de la tigelle S7. Chaque broche S5 est en outre pourvue d'une bague rotative S9 qui est engagée autour de la tigelle S7 en prenant appui sur l'embase S5. L'extrémité libre filetée S8 fait saillie hors de la bague rotative S9 afin de permettre un vissage de l'implant I sur l'extrémité libre filetée en venant en butée sur la bague rotative S9, comme cela est représenté sur la figure 2c. On peut clairement voir que l'extrémité libre filetée S8 est en prise avec le filetage interne I4 du logement creux I3 de l'implant I. Il suffit que l'implant I soit à peine vissé sur l'extrémité libre filetée S8 pour garantir son maintien sur la tigelle S7 en appui sur la bague rotative S9. Les éléments de support S avec les implants I montés dessus, comme représentés sur la figure 2b, présentent une configuration globale de peigne avec des dents constituées par des broches S5 sur lesquelles sont montés les implants I.

Les bagues rotatives S9 constituent selon l'invention un système de retrait pour retirer les implants des moyens de réception de l'élément de support constitué par les extrémités libres filetées S8. En effet, en entraînant les bagues rotatives S9 en rotation libre autour des tigelles S7, il s'ensuit un desserrage et un dévissage des implants I des extrémités supérieures filetées S8, et ce, sans qu'il soit nécessaire de venir en contact avec les implants I, et notamment avec leur partie exposée. En d'autres termes, les bagues rotatives permettent le dévissage des implants sans avoir à les toucher. Il est avantageux que les bagues rotatives S9 soient parfaitement alignées de sorte qu'une action commune et simultanée est possible sur l'ensemble des bagues rotatives pour dévisser simultanément tous les implants I d'un élément de support d'implants S. On peut par exemple imaginer une baguette rectiligne qui est mise en contact appuyé de l'ensemble des bagues rotatives S9 et à laquelle on imprime un mouvement rapide de va-et-vient, permettant d'assurer le dévissage des implants I. Les implants I peuvent alors tomber par gravité dans un récipient de récupération. Il va de soi que cette opération de retrait des implants I ne se fera qu'à la fin du procédé de greffage, après l'étape n de séchage.

A la place des bagues rotatives, on peut imaginer un autre système de retrait qui met par exemple en oeuvre un serrage par friction sollicité par des moyens élastiques pour défaire le serrage, par exemple par vibration. On peut aussi envisager de coller l'implant sur la broche avec un adhésif qui se défait lorsque soumis à des vibrations, de la chaleur, à un produit solvant, etc. Pour maintenir/retirer les implants, on peut également prévoir d'utiliser l'attraction/répulsion magnétique.

Sans sortir du cadre de l'invention, on peut également imaginer que les implants I soient dépourvus de filetage interne I4 et que la barrette S1 et les broches S5 soient creuses de manière à pouvoir générer une dépression qui va plaquer les implants I sur les extrémités libres des tigelles S7. La dépression peut par exemple être créée au niveau de l'extrémité de montage S4. Dans ce cas, il n'est pas nécessaire de prévoir des bagues rotatives S9. L'arrêt de la dépression constitue également un système de retrait. Toutefois, l'application d'une dépression nécessite une mise en oeuvre plus compliquée, c'est pourquoi l'utilisation de bagues rotatives S9 est une mise en oeuvre plus simple et efficace.

En se référant à la figure 3, on peut voir trois éléments de support S montés sur une platine de montage B comprenant plusieurs logements de montage B1 dans lesquels les extrémités de montage S4 des éléments de support S sont engagées et avantageusement bloquées aux moyens de vis de blocage B2. La figure 3 représente également un quatrième élément de support S prêt à être engagé dans son logement de montage B1 encore libre. Ainsi, quatre éléments de support S peuvent être montés sur une platine de montage avec une disposition mutuelle tête bêche. En effet, les deux éléments de support S les plus hauts sont disposés avec leurs broches S5 dirigées vers le bas, alors que les deux autres éléments de support S montés en bas sont disposés avec leurs broches S5 orientées vers le haut. Selon l'invention, la platine de montage B est pourvue d'un manche de préhension amovible B4 qui permet une manipulation aisée de la platine de montage B avec ses éléments de support S montés dessus.

On peut notamment utiliser la platine de montage B avec son manche amovible B4 lors des étapes a- à e- du procédé de greffage de polymère bioactif. On peut même se servir de la platine de montage B avec son manche amovible B4 pour disposer la platine de montage B avec ses éléments de support S dans le récipient rempli de gaz inerte tel que de l'argon. Ceci est visible sur la figure 4. Le manche amovible B4 doit alors être retiré laissant apparaître son logement de montage B3 au niveau de la platine B. Le récipient R peut être obturé à l'aide d'un couvercle R1 : ceci est toutefois optionnel, car l'argon est plus lourd que l'air et reste dans le récipient R même en l'absence de couvercle. Ainsi, la platine de montage B avec ses quatre éléments de support S sont introduits à l'intérieur de l'enceinte de polymérisation E qui est remplie de gaz inerte, tel que de l'argon, en étant disposé dans le récipient R. A l'intérieur de l'enceinte E, le couvercle R1 peut être retiré et la platine de montage B avec ses éléments de support S peut être extraite du récipient R.

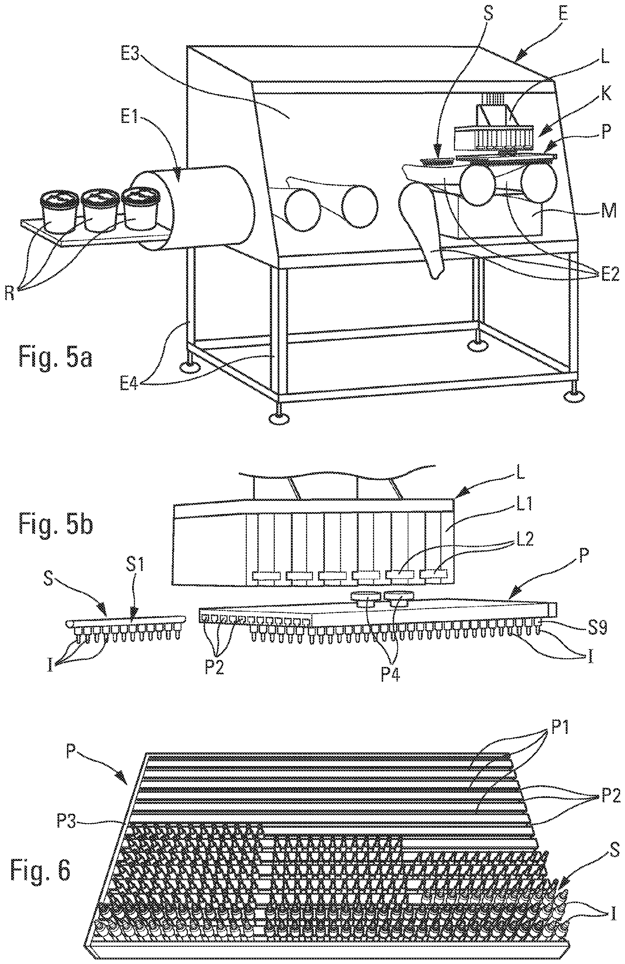

L'enceinte de polymérisation E est visible sur la figure 5a. Ce type d'enceinte est communément désigné sous l'expression « boite à gants », en raison de la présence de gants qui permettent d'effectuer des manipulations à l'intérieur de l'enceinte E à travers une paroi vitrée E3. Bien que non représentée, l'enceinte E est pourvue de moyens de traitement de son atmosphère interne afin de garantir des conditions optimales de pression, de pureté, et/ou d'hygrométrie. Le gaz le plus couramment utilisé est l'argon, bien que d'autres gaz peuvent également être mis en oeuvre. L'enceinte E est montée sur un piétement E4 et comprend un sas d'entrée E1 à travers lesquels les récipients R passent pour parvenir à l'intérieur de l'enceinte E. Le sas E1 comprend de manière conventionnelle une porte d'entrée et une porte de sortie afin de pouvoir contrôler l'atmosphère qui règne à l'intérieur du sas E1.

L'enceinte de polymérisation E contient une station de polymérisation K au niveau de laquelle les implants I sont plongés dans un bain de polymérisation pour permettre la synthèse de polymères bioactifs (par exemple du PolyNaSS) à la surface anodisée des implants, à partir d'un monomère X approprié, tel que du styrène sulfonate de sodium (NaSS). Cette station de polymérisation K comprend un ascenseur L qui est déplaçable verticalement au-dessus d'une cuve T qui est remplie de monomère X pour plonger/extraire les implants I dans et hors du bain de polymérisation de la cuve T. Cette station de polymérisation K comprend avantageusement des moyens catalyseurs pour accélérer la polymérisation sur les implants plongés dans le bain. Ces moyens catalyseurs peuvent se présenter sous la forme d'un bac de bain-marie M rempli de liquide O qui est chauffé par des moyens de chauffage M1, comme visible sur la figure 6. Les moyens catalyseurs peuvent également prendre la forme d'une source de rayonnement UV, comme on le verra ci-après.

En se référant simultanément aux figures 5b, 6 et 7 , on peut voir que l'ascenseur L comprend un chariot mobile verticalement L1 qui est monté sur une crémaillère verticale L4 de manière à permettre le déplacement vertical de haut en bas et de bas en haut du chariot L1. Ce chariot mobile est pourvu de moyens de montage aptes à recevoir une structure de support d'implants, comme on va le voir ci-après. Ces moyens de montage peuvent par exemple comprendre plusieurs glissières L2 dans lesquelles des moyens de fixation P4 de la structure de support sont insérables par coulissement. Les glissières L2 forment des ouvertures d'accès sur une face frontale tournée vers les gants de manipulation E2. L'ascenseur L peut optionnellement être pourvu d'un couvercle L3 qui vient coiffer, avantageusement de manière étanche, à la fois la cuve T remplie de monomère X et le bac de bain-marie M rempli de liquide chauffé O.

Dans le cas où le procédé de greffage de l'invention est appliqué à des implants dentaires, et plus particulièrement à des éléments de support S tels que décrits précédemment, il est prévu une plaque de montage P comprenant, sur une de ses faces, plusieurs rails de montage P1 dans lesquels les barrettes S1 des éléments de support S sont engagés par coulissement à partir d'une extrémité ouverte d'accès P2, comme cela est visible sur la figure 6 . Les éléments de support S peuvent ainsi être engagés les uns derrière les autres et les uns à côté des autres dans les rails de montage P1 de la plaque de montage P jusqu'à ce qu'elle soit pleine. Les broches S5 avec leurs implants montés dessus font saillie hors des rails de montage P1. La plaque de montage P est alors renversée et elle peut être montée dans les glissières L2 du chariot L1 au moyen d'un ou de plusieurs talon(s) de fixation P4. De cette manière, la plaque de montage P est fixée et disposée en dessous du chariot L1. L'ascenseur L peut alors être abaissé de manière à plonger les implants I dans le bain de monomères X. Le temps de polymérisation varie de 2 à 15 heures en fonction des moyens catalyseurs utilisés.

Selon un détail de manipulation, les éléments de support S sont retirés de la platine de support B à l'intérieur de l'enceinte E pour être montés individuellement dans les rails de montage P1 de la plaque de support P.

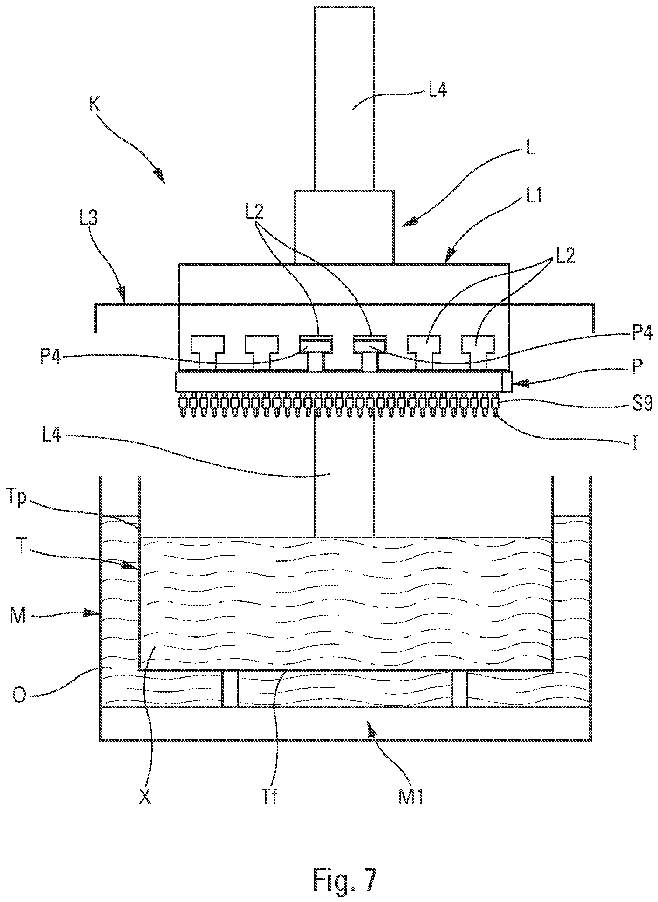

La cuve T de la figure 7, qui contient le monomère X, peut par exemple présenter une forme parallélépipédique avec un fond plat Tf et quatre parois latérales Tp. Avec des moyens catalyseurs sous la forme d'un bac de bain marie M, cette forme de réalisation est adéquate. Afin d'uniformiser la température à l'intérieur du bac de la cuve T, on peut prévoir des moyens générateurs de courant, comme par exemple un agitateur.

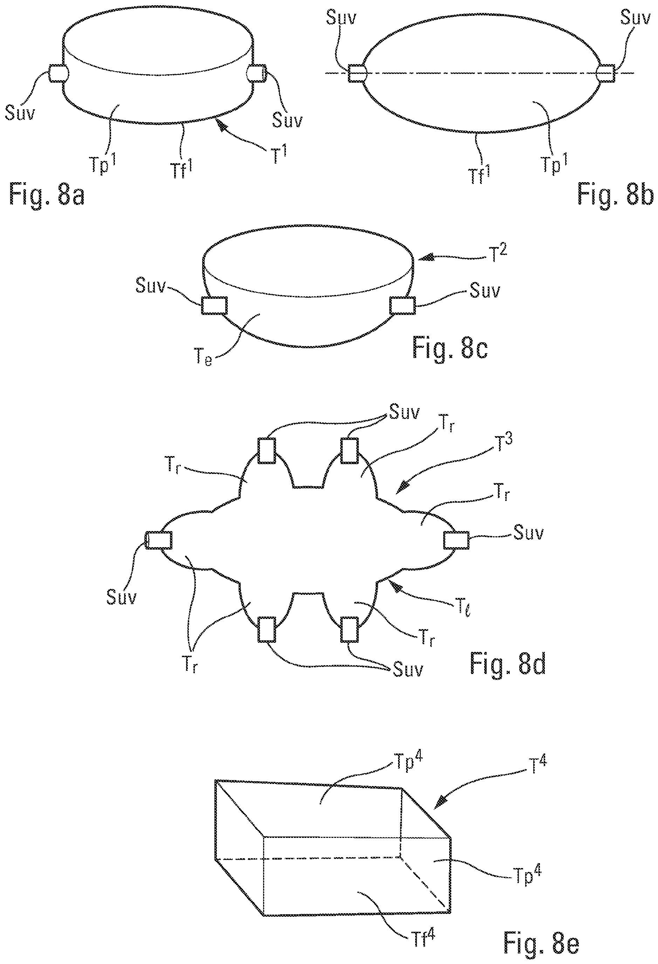

Les figures 8a à 8e illustrent de manière très schématique plusieurs variantes de réalisation pour une cuve remplie de monomère X dont les moyens catalyseurs se présentent sous la forme de sources de rayonnement UV. Sur les figures 8a et 8b, la cuve T¹ présente un fond plat Tf¹, mais une paroi latérale Tp¹ de forme elliptique. Deux sources de rayonnement UV Suv sont disposées l'une en face de l'autre sur le grand axe de l'ellipse, comme représenté sur la figure 8b. La forme elliptique de la paroi latérale Tp¹ permet d'améliorer la propagation des rayonnements UV à travers le monomère, particulièrement lorsque la paroi latérale Tp¹, et éventuellement aussi le fond Tf¹, sont réfléchissants. On peut par exemple prévoir une cuve T¹ en inox avec un fini miroir. On peut également prévoir un revêtement réfléchissement argenté à l'intérieur de la cuve T¹.

La figure 8c représente une variante dans laquelle la cuve T² présente une paroi Te en forme de coupelle ou d'auge, sans fond plat. Elle peut par exemple correspondre à une demie ellipsoïde de révolution. Les sources de rayonnement UV S_{UV} sont également disposées l'une en face de l'autre sur le grand axe de l'ellipsoïde, comme dans le mode de réalisation des figures 8a et 8b.

La figure 8d représente encore une autre variante de réalisation dans laquelle la cuve T³ est formée de plusieurs réflecteurs elliptiques Tr raccordés les uns aux autres par des tronçons de liaison Tl. La paroi latérale de la cuve T³ présente ainsi une forme complexe. Chaque réflecteur Tr est pourvu d'une source de rayonnement UV S_{UV}. La paroi latérale peut être réfléchissante, avec un fini miroir ou un revêtement argenté.

Sur la figure 8e représente une autre variante dans laquelle la cuve T⁴ est de forme parallélépipédique avec un fond plat Tf⁴ et les parois latérales Tp⁴. Une ou plusieurs de ces parois peuvent être constituées d'un panneau UV. On peut même imaginer que toute la cuve T⁴ soit constituée de cinq panneaux UV raccordés les uns autres aux autres.

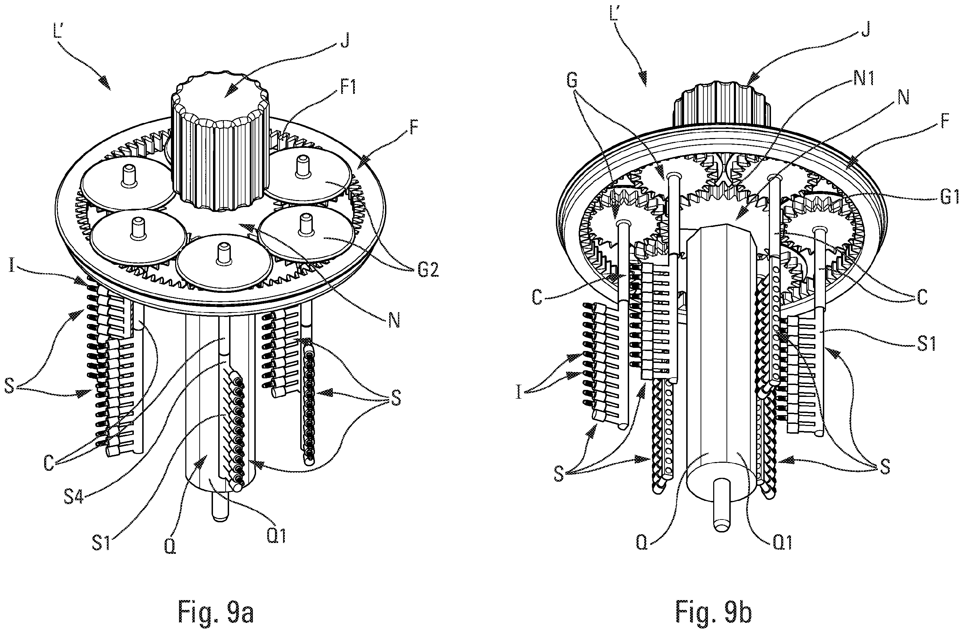

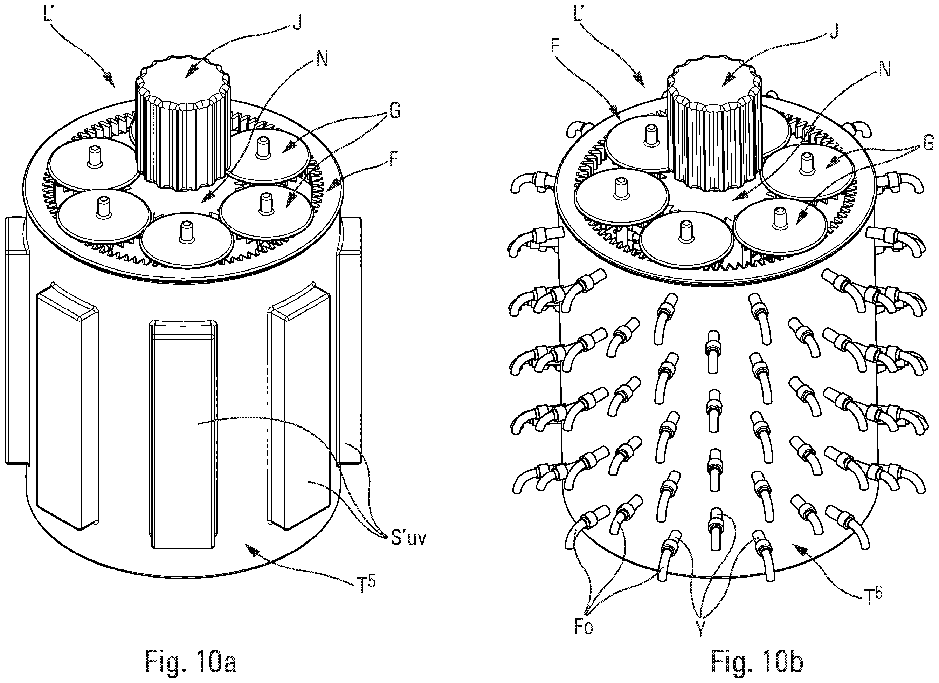

On se référera aux figures 9a, 9b, 10a et 10b pour décrire un second mode de réalisation pour un chariot L1' utilisable dans le cadre de la présente invention. Ce chariot L1' peut être mis en oeuvre à la place du chariot L1 précédemment décrit, qui est disposé à l'intérieur de l'enceinte étanche E. Alors que les implants I sont statiques dans la cuve lorsque le chariot L1 est dans sa position basse, les implants I montés sur le chariot L1 ' sont déplaçables à l'intérieur de la cuve, lorsque le chariot L1' est en position basse. En se référant aux figures 10a et 10b, on peut voir que les deux versions de cuves T⁵ et T⁶ présentent une forme sensiblement cylindrique avec des sources de rayonnement UV réparties sur sa périphérie. La cuve T⁵ comprend ainsi huit sources UV S'uv qui sont réparties de manière équidistante tout autour de la cuve. Les sources S'uv peuvent présenter une étendue axiale importante. Quant à la cuve T⁶, elle est pourvue d'une pluralité d'embouts optiques Y auxquels sont raccordées des fibres optiques Fo reliées à une source UV (non représentée). Compte tenu du nombre important d'embouts Y et de fibres optiques Fo, l'intérieur de la cuve T⁶ est irradiée de manière parfaitement homogène. La paroi interne de ces cuves T⁵ et T⁶ peuvent être réfléchissantes pour le rayonnement UV.

Sur les figures 10a et 10b, le chariot L1' est représenté dans sa position basse, dans lequel il est introduit au maximum à l'intérieur des cuves T⁵ et T⁶. Sur ces figures, il n'est pas représenté de quelle manière le chariot L1' est déplacé axialement verticalement, mais n'importe quel moyen approprié peut être utilisé.

On se référera maintenant aux figures 9a et 9b pour décrire en détail la structure et le fonctionnement de ce chariot L1'. Il est ici destiné à recevoir des éléments de support S tels que décrits précédemment, mais il peut également être envisageable que des platines de support B dotées de plusieurs éléments de support S soient reçus par le chariot L1'. Dans le cas particulier des figures 9a et 9b, chaque élément de support S est monté par son extrémité de montage S4 à l'extrémité libre d'une tige axiale verticale C qui forme l'axe de rotation d'une roue horizontale G. Chaque roue G est dotée d'un disque supérieur G1 à travers lequel débouche l'extrémité supérieure de la tige axiale verticale C. Sur les figures 9a et 9b, les roues horizontales G sont en nombre de six, mais ce nombre n'est pas limitatif. Ces roues horizontales G sont disposées à l'intérieur d'une couronne de révolution F qui est dentée intérieurement en F1. Les dents G1 des roues horizontales G sont en prise avec les dents F1 de la couronne de révolution F. Le disque G2 repose avec leur bord externe sur la couronne de révolution F. D'autre part, les roues dentées G sont également en prise avec une roue d'entraînement centrale N qui est également dentée en N1. Cette roue centrale N est pourvue en partie haute d'un plot d'entraînement J destiné à venir en prise avec des moyens d'entraînement en rotation (non représentés). A son extrémité inférieure, la roue d'entraînement N est pourvue d'un moyeu Q qui s'étend entre les tiges axiales verticales C et les éléments de support S. Avantageusement, le moyeu Q est réfléchissant, tout particulièrement aux rayonnements UV. On peut par exemple réaliser le moyeu Q avec des facettes réfléchissantes Q1. Bien que non représenté, le chariot L1' peut être pourvu d'un couvercle qui coiffe hermétiquement les roues horizontales G et la couronne de révolution F.

Avec une telle conception, on comprend aisément que l'entraînement en rotation du plot d'entraînement J a pour effet d'entraîner la roue d'entraînement N en rotation sur elle-même. Etant donné que la roue d'entraînement N est en prise engrenée avec les roues horizontales G, ces dernières sont entraînées en rotation à la fois sur elle-même, mais également autour de la roue d'entraînement N à l'intérieur de la couronne de révolution F. Par conséquent, les éléments de support S sont entraînés à la fois en rotation autour d'un axe passant par les tiges C et leurs barrettes S1, mais également en rotation de révolution autour d'un axe central passant par le plot d'entraînement J, la roue d'entraînement N et le moyeu Q. Les éléments de support S effectuent donc un mouvement complexe résultant de la combinaison d'une rotation autour de leurs barrettes et d'une révolution autour de la roue d'entraînement centrale N. De cette manière, les implants I montés sur les éléments de support S se déplacent selon une trajectoire complexe à l'intérieur du monomère présent dans la cuve. On garantit ainsi que les implants I sont exposés de manière identique et homogène au rayonnement UV qui irradie le monomère présent dans la cuve.

Avec un ascenseur L' associé à une cuve irradiée par un rayonnement UV, comme les cuves T⁵ et T⁶, on obtient un revêtement greffé de polymère bioactif, tel que du PolyNaSS, avec une épaisseur et une densité souhaitées. Le temps nécessaire pour une polymérisation satisfaisante peut être considérablement réduit par rapport à une polymérisation avec catalyseur thermique (du type bain-marie) qui est de l'ordre de 15 heures. On peut en effet réduire aisément ce temps de moitié, et même davantage avec un rayonnement UV et une cuve optimisés, pour atteindre un temps de l'ordre de 2 à 5 heures, voire encore moins de l'ordre de 1 heure.

Une fois le greffage achevé, les implants sont extraits de la cuve en remontant le chariot L1 ou L1'. Les éléments de support S peuvent alors être retirés du chariot en les manipulant à l'aide des gants de manipulation E2. Ils peuvent être ainsi amenés dans le sas d'entrée E1, d'où ils sont extraits de l'enceinte étanche E. Ils peuvent ensuite subir les étapes de lavage pour les débarrasser de l'excès de polymère bioactif greffé et de séchage.

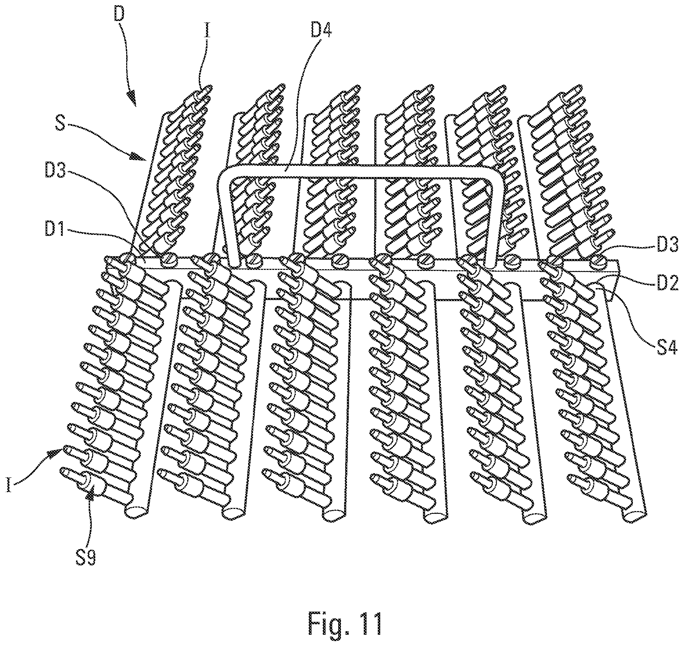

En se référant à la figure 11, on peut voir un autre ustensile ou ancillaire qui permet une manipulation avantageuse des éléments de support S lors des étapes de lavage et de séchage. Cet ustensile peut être qualifié de panier de lavage D, étant donné qu'il présente une configuration proche de celle des paniers que l'on rencontre dans les lave-vaisselles. Ce panier de lavage D comprend une poutre centrale D1 qui forme plusieurs logements de montage D2 dans lesquels sont engagées les extrémités de montage S4 des éléments de support S. Des vis de blocage optionnels D3 peuvent être prévus pour bloquer les extrémités de montage S4 à l'intérieur des logements de montage D2. La poutre centrale D1 peut également être pourvue d'une anse de préhension D4 par laquelle on peut saisir manuellement le panier de lavage D. Sur la figure 11, on peut dénombrer douze éléments de support S, à savoir six de chaque côté de la poutre centrale D1. On peut remarquer que les éléments de support S sont disposés avec une orientation inclinée, de l'ordre de 45° par rapport à l'horizontale ou la verticale. L'inclinaison des éléments de support S de part et d'autre de la poutre centrale D1 sont opposés, ou décalés de 90°. En effet, les implants I visibles en dessous de la poutre centrale D1 sur la figure 11 sont orientés vers la gauche, alors que les implants I disposés au-dessus de la poutre centrale D1 sont orientés vers la droite.

Ce panier de lavage D peut ainsi aisément être disposé dans un équipement de lavage approprié, dans lequel de l'eau, de préférence hautement purifiée, est projetée par aspersion sur les implants I, afin de les débarrasser de leur excès de polymère bioactif greffé. Cette phase de lavage est suivie par une phase de séchage qui peut être opérée dans le même appareil de lavage.

A la fin de l'étape de séchage, le panier D est extrait de l'appareil de lavage/séchage, et les éléments de support S sont retirés de la poutre centrale D1. Ensuite, les implants I peuvent être retirés de l'élément de support S, comme précédemment expliqué, en entraînant les bagues rotatives S9 en rotation, afin de dévisser les implants I des extrémités filetées S8. Sans sortir du cadre de l'invention, on peut également imaginer que les implants I soient maintenus par aspiration sur des éléments de support appropriés.

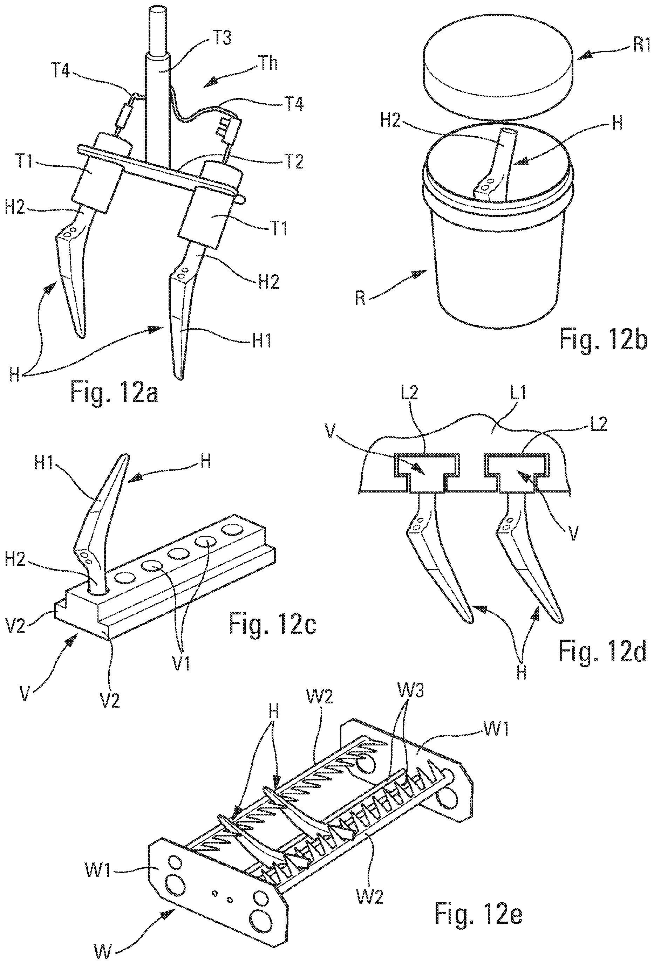

Jusqu'à présent, toute la description a été faite en référence à un type d'implant particulier, à savoir les implants dentaires I. L'invention n'est toutefois pas limitée à ce type d'implant particulier, et d'autres types d'implants peuvent être traités, revêtus et manipulés selon l'invention. En se référant aux figures 12a à 12f, il est fait référence à un autre type d'implant, à savoir un implant fémorale de hanche H. On peut également parler de prothèse fémorale. Cet insert H comprend de manière typique une broche fémorale H1 destinée à être insérée et scellée dans le fémur et une tige de col H2 destinée à recevoir la tête fémorale qui est reçue dans la cotyle fixé au bassin. Les prothèses fémorales H ici traitées sont dépourvues de têtes.

En se référant à la figure 12a, on peut voir un premier dispositif de support sous la forme d'un étrier Th qui permet de recevoir deux implants fémoraux H. Plus précisément, cet étrier Th comprend deux manchons de réception T1 dans lesquels sont reçues respectivement les tiges de col H2 des implants H. Ces manchons T1 sont montés sur une plaquette commune qui est pourvue d'une tige de montage T3. Les deux manchons T1 sont également pourvus de moyens de câblage électriques T4 pour alimenter les inserts H en courant. Cet étrier Th peut être utilisé lors des étapes b, c, d, et e du procédé de greffage. Cet étrier Th constitue une structure de support d'implants, au même titre que l'élément de support S, la platine de support B et la plaque de support précédemment décrits. L'étrier Th peut être monté sur un ascenseur au moyen de sa barre de montage T3 pour réaliser les étapes de décapage, d'anodisation et de rinçage.

Une fois ces étapes terminées, les implants H peuvent être retirés de l'étrier Th et placés individuellement ou en groupe dans un récipient R, tel que celui utilisé précédemment pour les implants dentaires. Ce récipient R est avantageusement rempli d'un gaz inerte, tel que de l'argon, et pourvu optionnellement d'un couvercle R1. Les implants peuvent ainsi être introduits dans l'enceinte de polymérisation E à travers le sas d'entrée E1. A l'intérieur de l'enceinte, les implants H peuvent être montés sur une réglette V, visible sur la figure 12c. Cette réglette V comprend des trous de réception V1 dans lesquels les tiges de col H2 sont insérées en force. La réglette V comprend également deux brides longitudinales opposées V2, qui vont permettre l'insertion par coulissement des réglettes V dans les glissières L2 du chariot L1 de l'ascenseur L, comme visible sur la figure 12d. Les implants H peuvent ainsi être plongés dans la cuve remplie de monomère X. Après un temps de polymérisation suffisant, les implants peuvent être extraits de la cuve et les réglettes V peuvent être extraites des glissières L2. Les implants H peuvent ensuite être retirés des réglettes V et extraits de l'enceinte étanche E à travers le sas d'entrée. En variante, les implants H peuvent être montés sur les tiges axiales verticales C de l'ascenseur L'. Il est également envisageable de monter plusieurs implants H sur une même tige axiale verticale C en utilisant une structure de support, tel que l'étrier Th.

Pour les étapes de lavage et de séchage ultérieures, il est prévu un casier de lavage W (figure 12e) sur lequel les implants greffés peuvent être déposés. Ce casier de lavage W comprend deux flasques d'extrémité W1 reliées entre elles par deux râteaux de rangement W2 et deux tiges de support W3. Les implants H peuvent ainsi être disposés entre les dents des râteaux W2 et en appui sur les tiges de support W3.

Il est à noter que certains aspects particuliers liés à la manipulation des implants fémoraux H peuvent être protégés indépendamment des autres caractéristiques déjà exposées et pourraient de ce fait faire l'objet de demandes divisionnaires. Plus particulièrement, l'étrier Th, la réglette V et le casier de lavage W pourraient faire l'objet de dépôts de brevet en soi.

Grâce à l'invention, on dispose d'un procédé de greffage de polymère bioactif sur des implants qui peut mis en oeuvre de manière industrielle à très grande échelle. Les ustensiles qui ont été conçus, tels que l'élément de support S, la platine de support B, la plaque de support P, le casier de lavage D, l'étrier de support Th, la réglette V et le casier de lavage W permettent d'optimiser le procédé de greffage sur le plan industriel. Enfin, l'enceinte étanche E, et plus particulièrement son chariot et sa cuve rendent possibles une industrialisation à grande échelle de l'étape de polymérisation. Des implants, notamment dentaires et de hanche, peuvent ainsi être revêtus avec un polymère bioactif, tel que du PolyNaSS, à grande échelle et à grande cadence.

## Revendications

1. Elément de support (S) d'implants (I), tels que des implants dentaires, comprenant une barrette (S1) et plusieurs broches (S5) raccordées à la barrette (S1) et disposées parallèlement les unes aux autres, chaque broche (S5) définissant une extrémité libre pourvue de moyens de réception (S8) aptes à coopérer avec l'implant (S) pour le maintenir sur les moyens de réception (S8) de la broche (S5), la barrette (S1) comprenant au moins une extrémité de montage (S4) pour le montage de la barrette (S1) sur un autre dispositif de support pour ainsi former une structure de support,
**caractérisé en ce que** chaque broche (S5) est pourvue d'un système de retrait (S9) pour retirer l'implant (S) des moyens de réception (S8) sans venir en contact avec une partie exposée de l'implant (S).

2. Elément de support (S) selon la revendication 1, dans lequel le système de retrait comprend une bague rotative (S9) montée sur la broche (S5), l'implant (S) en prise avec les moyens de réception (S8) venant en contact de la bague rotative (S9), de sorte qu'une rotation de la bague rotative (S9) a pour effet de désolidariser l'implant (S) de ses moyens de réception (S8).

3. Elément de support (S) selon la revendication 2, dans lequel chaque broche (S5) comprend une embase (S6) raccordée à la barrette (S1), l'embase (S6) étant surmontée d'une tigelle (S7) dont l'extrémité libre est pourvue d'un filetage (S8) formant les moyens de réception, la bague rotative (S9) étant engagée autour de la tigelle (S7) en prenant appui sur l'embase (S6), le filetage (S8) faisant saillie hors de la bague rotative (S9) afin de permettre un vissage de l'implant (I) sur le filetage (S8) en venant en butée sur la bague rotative (S9).

4. Elément de support (S) selon l'une quelconque des revendications précédentes, dans lequel la barrette (S1) supporte une douzaine de broches (S5) avec un écart entre broches de l'ordre de 1 à 2 cm.

5. Elément de support (S) selon l'une quelconque des revendications précédentes, dans lequel la barrette (S1) présente une section transversale de forme cylindrique circulaire tronquée de manière à former un méplat longitudinal (S3) qui est disposé de manière opposée aux broches (S5).

6. Platine de montage (B) d'éléments de support (S) d'implants (I), comprenant plusieurs logements de montage (B1) dans lesquels sont engagées les extrémités de montage (S4) des barrettes (S1) d'éléments de support (S) d'implants (I) selon l'une quelconque des revendications précédentes

7. Platine de montage (B) selon la revendication 6, dans laquelle les broches (S5) sont disposées tête-bêche les unes par rapport aux autres.

8. Platine de montage (B) selon la revendication 6 ou 7, comprenant un manche de préhension amovible (B4).

9. Plaque de montage (P) d'éléments de support (S) d'implants (I), comprenant plusieurs rails de montage (P1) dans lesquels sont engagées les barrettes (S1) d'éléments de support (S) d'implants (I) selon l'une quelconque des revendications 1 à 5.

10. Plaque de montage (P) selon la revendication 9, dans laquelle les barrettes (S1) sont engagées par coulissement à partir d'une extrémité ouverte d'accès (P2) des rails de montage (P1), les broches (S1) pointant parallèlement dans la même direction.

11. Plaque de montage (P) selon la revendication 9 ou 10, comprenant des moyens de fixation (P4) disposés sur une face opposée aux rails de montage (P1) pour fixer la plaque de montage (P) sur un chariot d'élévateur (L).

12. Panier de lavage (D) comprenant une poutre centrale (D1) formant des logements de montage (D2) dans lesquels sont engagées les extrémités de montage (S4) des barrettes d'éléments de support (S) d'implants (I) selon l'une quelconque des revendications 1 à 5.

13. Panier de lavage (D) selon la revendication 12, dans lequel les éléments de support (S) d'implants (I) sont disposés de part et d'autre de la poutre centrale (D1) de manière équilibrée, avantageusement avec une inclinaison.

14. Panier de lavage (D) selon la revendication 12 ou 13, dans lequel la poutre centrale (D1) est pourvue d'une anse de préhension (D4).

## Patentansprüche

1. Trägerelement (S) für Implantate (1), beispielsweise Zahnimplantate, umfassend eine Stange (S1) und mehrere mit der Stange (S1) verbundene und parallel zueinander angeordnete Stifte (S5), wobei an jedem Stift (S5) ein freies Ende ausgebildet ist, das mit Aufnahmemitteln (S8) versehen ist, die mit dem Implantat (S) zusammenwirken können, um es an den Aufnahmemitteln (S8) des Stifts (S5) zu halten, wobei die Stange (S1) mindestens ein Befestigungsende (S4) zum Befestigen der Stange (S1) an einer anderen Haltevorrichtung umfasst, wodurch eine Trägerstruktur gebildet wird, **dadurch gekennzeichnet, dass** jeder Stift (S5) mit einem Entfernsystem (S9) versehen ist, mit dem sich das Implantat (S) von den Aufnahmemitteln (S8) entfernen lässt, ohne dabei mit einem freiliegenden Teil des Implantats (S) in Kontakt zu kommen.

2. Trägerelement (S) nach Anspruch 1, bei dem das Entfernsystem einen am Stift (S5) angebrachten Drehring (S9) umfasst, wobei das mit den Aufnahmemitteln (S8) in Eingriff stehende Implantat (S) mit dem Drehring (S9) in Kontakt kommt, so dass sich infolge der Drehung des Drehrings (S9) das Implantat (S) von seinen Aufnahmemitteln (S8) löst.

3. Trägerelement (S) nach Anspruch 2, bei dem jeder Stift (S5) einen mit der Stange (S1) verbundenen Sockel (S6) umfasst, wobei der Sockel (S6) von einem Stift (S7) überragt wird, dessen freies Ende mit einem Gewinde (S8) versehen ist, das die Aufnahmemittel bildet, wobei der Drehring (S9) den Stift (S7) umgreift und sich dabei am Sockel (S6) abstützt, wobei das Gewinde (S8) aus dem Drehring (S9) herausragt, um ein Aufschrauben des Implantats (1) derart auf das Gewinde (S8) zu ermöglichen, dass es dabei am Drehring (S9) in Anlage kommt.

4. Trägerelement (S) nach einem der voranstehenden Ansprüche, bei dem die Stange (S1) ein Dutzend Stifte (S5) trägt, wobei der Abstand zwischen den Stiften in der Größenordnung von 1 cm bis 2 cm liegt.

5. Trägerelement (S) nach einem der voranstehenden Ansprüche, bei dem die Stange (S1) eine abgestumpft-kreiszylindrische Querschnittsform aufweist, so dass sie eine längsseitig verlaufende Abflachung (S3) ausbildet, welche den Stiften (S5) gegenüberliegend angeordnet ist.

6. Montageplatine (B) für Trägerelemente (S) für Implantate (I), umfassend mehrere Montageaufnahmen (B1), in die die Befestigungsenden (S4) der Stangen (S1) von Trägerelementen (S) für Implantate (I) nach einem der voranstehenden Ansprüche eingreifen.

7. Montageplatine (B) nach Anspruch 6, bei der die Stifte (S5) entgegengesetzt zueinander liegend angeordnet sind.

8. Montageplatine (B) nach einem der Ansprüche 6 oder 7, umfassend einen abnehmbaren Greifgriff (B4).

9. Montageplatine (P) für Trägerelemente (S) für Implantate (I), umfassend mehrere Montageschienen (P1), in die die Stangen (S1) der Trägerelemente (S) für Implantate (I) nach einem der Ansprüche 1 bis 5 eingreifen.

10. Montageplatine (P) nach Anspruch 9, bei der die Stangen (S1) durch Einschieben über ein zugängliches offenes Ende (P2) der Montageschienen (P1) eingeführt werden, wobei die Stangen (S1) parallel in die gleiche Richtung zeigen.

11. Montageplatine (P) nach Anspruch 9 oder 10, mit Befestigungsmitteln (P4), die auf einer den Montageschienen (P1) gegenüberliegenden Seite angeordnet sind, um die Montageplatine (P) an einem Hubwagen (L) zu befestigen.

12. Spülkorb (D) mit einem Mittelträger (D1), an dem Montageaufnahmen (D2) ausgebildet sind, in denen die Befestigungsenden (S4) der Stangen der Trägerelemente (S) für Implantate (I) nach einem der Ansprüche 1 bis 5 aufgenommen werden.

13. Spülkorb (D) nach Anspruch 12, bei dem die Trägerelemente (S) für Implantate (I) auf beiden Seiten des Mittelträgers (D1) gleichmäßig, vorzugsweise mit einer Neigung, angeordnet sind.

14. Spülkorb (D) nach Anspruch 12 oder 13, bei dem der Mittelträger (D1) mit einer Griffbügel (D4) versehen ist.

## Claims

1. A support element (S) for supporting implants (I), such as dental implants, the support element comprising a bar (S1) and a plurality of pins (S5) that are fitted to the bar (S1) and that are arranged parallel to one another, each pin (S5) defining a free end that is provided with reception means (S8) that are suitable for co-operating with the implant (S) so as to hold it on the reception means (S8) of the pin (S5), the bar (S1) including at least one mounting end (S4) for mounting the bar (S1) on another support device, thereby forming a support structure;
the support element being **characterized in that** each pin (S5) is provided with a removal system (S9) for removing the implant (S) from the reception means (S8), without coming into contact with an exposed portion of the implant (S).

2. A support element (S) according to claim 1, wherein the removal system comprises a rotary ring (S9) that is mounted on the pin (S5), the implant (S) in engagement with the reception means (S8) coming into contact with the rotary ring (S9), such that one revolution of the rotary ring (S9) causes the implant (S) to disengage from its reception means (S8).

3. A support element (S) according to claim 2, wherein each pin (S5) includes a base (S6) that is fitted to the bar (S1), the base (S6) being surmounted by a rod (S7) having a free end that is provided with a thread (S8) forming the reception means, the rotary ring (S9) being engaged around the rod (S7), bearing against the base (S6), the thread (S8) projecting out from the rotary ring (S9) in order to enable the implant (I) to be screw-fastened on the thread (S8), coming into abutment against the rotary ring (S9).

4. A support element (S) according to any preceding claim, wherein the bar (S1) supports about twelve pins (S5) with the pins at a spacing that lies in the range about 1 cm to 2 cm.

5. A support element (S) according to any preceding claim, wherein the bar (S1) presents a cross-section of circularly-cylindrical shape that is truncated in such a manner as to form a longitudinal flat (S3) that is arranged remote from the pins (S5).

6. A mounting slab (B) for mounting support elements (S) for supporting implants (I), the mounting slab including a plurality of mounting housings (B1) receiving the mounting ends (S4) of the bars (S1) of support elements (S) for supporting implants (I) according to any preceding claim

7. A mounting slab (B) according to claim 6, wherein the pins (S5) are arranged opposite ways round relative to one another.

8. A mounting plate (B) according to claim 6 or claim 7, including a removable handle (B4).

9. A mounting plate (P) for mounting support elements (S) for supporting implants (I), the mounting plate including a plurality of mounting rails (P1) receiving the bars (S1) of support elements (S) for supporting implants (I) according to any one of claims 1 to 5.

10. A mounting plate (P) according to claim 9, wherein the bars (S1) are engaged by sliding into an open access end (P2) of the mounting rails (P1), the pins (S1) being parallel and pointing in the same direction.

11. A mounting plate (P) according to claim 9 or claim 10, including fastener means (P4) that are arranged on a face remote from the mounting rails (P1) for fastening the mounting plate (P) on an elevator carriage (L).

12. A washing rack (D) comprising a central pole (D1) that forms mounting housings (D2) that receive the mounting ends (S4) of the bars of support elements (S) for supporting implants (I) according to any one of claims 1 to 5.

13. A washing rack (D) according to claim 12, wherein the support elements (S) for supporting implants (I) are arranged on either side of the central pole (D1) in balanced manner, advantageously sloping.

14. A washing rack (D) according to claim 12 or claim 13, wherein the central pole (D1) is provided with a handle (D4).
